# EUROPÄISCHE PATENTSCHRIFT

(11) **EP 1 266 673 B1**
(45) Veröffentlichungstag und Bekanntmachung des Hinweises auf die Patenterteilung: **19.08.2009**
(21) Anmeldenummer: 02090205.2
(22) Anmeldetag: 06.06.2002
(51) Int. Cl.: A61N 1/37

(54) **Stimulationsgerät**
Stimulating device
Appareil de stimulation

(30) Priorität: 14.06.2001 DE 10129649
(43) Veröffentlichungstag der Anmeldung: 18.12.2002
(73) Patentinhaber: BIOTRONIK GmbH & Co. KG, 12359 Berlin (DE)
(72) Erfinder: Hutten, Helmut, Prof.Dr., A-8042 Graz (AT); Schaldach, Max, Prof.Dr., Verstorben (DE)
(74) Vertreter: Lindner-Vogt, Karin L.

(56) Entgegenhaltungen:
- US-A- 5 476 487
- US-A- 5 480 414
- US-A- 5 674 254

## Beschreibung

Die Erfindung betrifft ein Stimulationsgerät, insbesondere für ein menschliches Herz, mit einer Stimulationseinheit, die zum Abgeben eines Stimulationsimpulses mit einer Impulsdauer, einer Impulsstärke und einer Stimulationsintensität ausgebildet ist, mit einem Signaldetektor, der zum Detektierten eines Stimulationserfolges anhand eines aufgenommenen Signals ausgebildet ist und mit einer Steuereinheit, die mit der Stimulationseinheit und mit dem Signaldetektor derart verbunden und ausgebildet ist, dass die Stimulationsintensität in Abhängigkeit des aufgenommenen Signals veränderbar ist.

Zu den genannten Stimulationsgeräten zählen insbesondere auch implantierbare Herzschrittmacher. Diese sind üblicherweise über eine Elektrodenleitung mit einer in einem Herz angeordneten Elektrode verbunden und ausgebildet, über die Elektrode elektrische Stimulationsimpulse an das Herz abzugeben. Diese Stimulationsimpulse dienen der Erregung des Herzgewebes oder Myokards und werden je nach Herzschrittmacherart insbesondere dann abgegeben, wenn das Herz sich nicht auf natürliche Weise kontrahiert. In diesem Falle wird eine Kontraktion durch elektrisches Stimulieren des Herzgewebes hervorgerufen.

Ein solcher elektrischer Stimulationsimpuls muss eine Stimulationsintensität besitzen, die über einer jeweiligen Reizschwelle liegt. Die Reizschwelle ist dabei ein Maß für die Mindeststimulationsintensität, die ausreicht, um eine Dipolarisation des Myokards und damit einer Kontraktion einer jeweiligen Kammer des Herzens zu bewirken. Die Reizschwelle hängt von verschiedenen Faktoren ab und ist darüber hinaus im Laufe der Zeit unter Umständen auch veränderlich.

Neben dem Erfordernis, einen Stimulationsimpuls ausreichender Stimulationsintensität abzugeben, besteht das Bedürfnis, die für einen Stimulationsimpuls anzuwendende Energie so gering wie möglich zu halten. Diese Energie wird regelmäßig einer Batterie des Herzschrittmachers entnommen, die sich im Laufe der Zeit erschöpft. Wenn diese Batterie erschöpft ist, muss der Herzschrittmacher in einer Operation durch einen neuen ersetzt werden.

Es besteht also einerseits das Erfordernis, dass die Stimulationsintensität eines Stimulationsimpulses ausreichen muss, eine Kontraktion des Herzgewebes auszulösen. Die Stimulationsintensität hängt dabei einerseits von der Dauer eines Stimulationsimpulses und andererseits von der Stärke eines Stimulationsimpulses ab. Die Stärke eines Stimulationsimpulses wiederum hängt von der elektrischen Spannung ab, mit der ein Stimulationsimpuls an das Herzgewebe abgegeben wird. Dies bedeutet, dass mit einer größeren Stimulationsintensität regelmäßig auch ein größerer Energieverbrauch verbunden ist.

Andererseits besteht das Bedürfnis, den Energieverbrauch pro Stimulationsimpuls so gering wie möglich zu halten, da diese Energie einer Batterie des Herzschrittmachers entnommen wird, die auf diese Weise erschöpft wird. Wenn die Batterie des Herzschrittmachers erschöpft ist, ist einer Operation zum Austausch des Herzschrittmachers oder der Batterie erforderlich. Eine Senkung des Energieverbrauchs des Herzschrittmachers geht also mit einer höheren Lebensdauer des Herzschrittmachers einher.

Es besteht somit das Bedürfnis, die Anforderungen nach einer möglichst geringen Stimulationsintensität und gleichzeitig einer regelmäßig erfolgreichen Stimulation durch Optimieren der Stimulationsintensität zu erfüllen. Hierzu ist es aus dem Stand der Technik beispielsweise aus den US-Patenten 5,350,410; US 5,411,533; US 5,431,693 und US 5,674,254 bekannt, nach Abgabe eines Stimulationsimpulses den Stimulationserfolg (capture) zu erfassen (capture recognition) um zumindest bei mangelhaftem Stimulationserfolg einen Backup-Stimulationsimpuls auszulösen.

Aus US 5,480,414 ist ein Stimulationsgerät bekannt, welches prüft, ob ein Stimulationserfolg in ein vorgegebenes Zeitfenster nach Abgabe eines Stimulationsimpulses fällt.

Aus US 5, 476,487 ist ein Stimulationsgerät bekannt, welches immer paarweise Stimulationsimpulse mit einem Abstand von 60 ms bis 100 ms voneinander abgibt, von denen der erste Stimulationsimpuls eine variable Stimulationsintensität hat, während der zweite Stimulationsimpuls auf jeden Fall überschwellig ist. Führt bereits der erste der Stimulationsimpulse eines Paares zu einem Stimulationserfolg, ist der zweite Stimulationsimpuls unwirksam, weil zum Zeitpunkt seiner Abgabe das Myokard refraktär ist. Ist hingegen der erste Stimulationsimpuls unterschwellig und damit unwirksam, führt der zweite der beiden Stimulationsimpulse zu einem Stimulationserfolg. Der zeitliche Abstand zwischen Abgabe des ersten Stimulationsimpulses und Eintreten des Stimulationserfolges ändert sich sprunghaft je nachdem, ob der erste Stimulationsimpuls oder der zweite Stimulationsimpuls zu einem Stimulationserfolg geführt haben. Die Steuerung der Impulsstärke des ersten Stimulationsimpulses erfolgt in Abhängigkeit des Erkennens einer sprunghaften Änderung der Zeitdauer zwischen Abgabe des ersten Stimulationsimpulses und Eintreten des Stimulationserfolges.

Gegenüber den bekannten Herzschrittmachem mit capture recognition und Anpassung der Stimulationsintensität besteht insbesondere der Wunsch, einen mangelnden Stimulationserfolg so schnell wie möglich zu erkennen, um einen BackupStimulationsimpuls so schnell wie möglich auf den zuvor abgegebenen Stimulationsimpuls abgeben zu können.

Dem liegt das Problem einer zuverlässigen und schnellen Stimulationserfolgskontrolle zu Grunde.

Erfindungsgemäß wird dieses Problem durch ein Stimulationsgerät gemäß des Anspruchs 1 gelöst.

Der Erfindung liegt die Erkenntnis zu Grunde, dass die Zeitdifferenz zwischen Abgabe eines Stimulationsimpulses und Erfassen eines Stimulationserfolges ein Maß dafür ist, ob die Stimulationsintensität der Reizschwelle entspricht oder deutlich über dieser liegt, also überschwellig ist. Bei einer deutlich überschwelligen Stimulationsintensität ist die Zeitdifferenz zwischen Abgabe des Stimulationsimpulses und Erfassen des Stimulationserfolges bzw. Erfassen eines den Stimulationserfolg kennzeichnenden Signalmerkmals kürzer, als bei optimal angepasster Stimulationsintensität. Falls die Stimulationsintensität nicht ausreicht, ist hingegen möglicherweise überhaupt kein Stimulationserfolg erfassbar.

In dem allein die Zeit zwischen Stimulationsimpulsabgabe und erwartetem Stimulationserfolg als Maß einerseits für die Bestimmung der optimalen Stimulationsintensität und andererseits für die Kontrolle des Stimulationserfolges herangezogen wird, kann die Abgabe eines Backup-Stimulationsimpulses frühestmöglich erfolgen. Es braucht nämlich jeweils nur die Zeit abgewartet zu werden, nach der der Stimulationserfolg nach Abgabe eines Stimulationsimpulses spätestens zu erwarten ist um nach Ablauf dieser Zeit schnellstmöglich einen Backup-Impuls abzugeben. Tritt der Stimulationserfolg hingegen deutlich früher als zur erwarteten Zeit auf, kann die Stimulationsintensität schrittweise verringert werden.

Die von dem Stimulationsgerät erfasste Zeitdifferenz ist somit in idealer Weise ein Maß gleichzeitig für die Anpassung der Stimulationsintensität als auch für die Stimulationserfolgskontrolle.

Das Erhöhen oder Verringern der Stimulationsintensität erfolgt vorzugsweise dadurch, dass die Stimulationseinheit zwei Kondensatoren unterschiedlicher Ladung umfasst und zum Erhöhen oder Verringern der Stimulationsintensität auf den jeweils anderen Kondensator umgeschaltet wird. Im Falle eines Zweikammerschrittmachers können als die beiden Kondensatoren auch jeweils ein Kondensator einer atrialen und einer ventrikulären Stimulationseinheit vorgesehen sein, die entsprechend verschaltet sind.

Gemäß eines auch selbständig schutzwürdigen Gedankens sollen somit zwei Ladekondensatoren vorhanden sein, von denen einer in der üblichen Weise auf die aktuell zur erfolgreichen Stimulation erforderlich erscheinende Spannung und der zweite permanent oder nur kurzzeitig auf eine geringfügig niedrigere Spannung aufgeladen wird. Dann wird "versuchsweise" mit der niedrigeren Spannung (d.h. es wird auf den zweiten Ladekondensator zugegriffen) stimuliert. Wenn die Stimulation mit der geringeren Spannung nicht erfolgreich ist, wird unmittelbar nach Feststellung der Erfolglosigkeit mit der zuvor verwendeten höheren Spannung stimuliert. "Unmittelbar danach" heißt nach spätestens 50 ms. Spätestens nach 50 ms (in diese Periode fällt wesentlich das Autoshorting, aber dieses Intervall läßt sich noch verkürzen, grundsätzlich gestatten fraktale Elektroden eine Feststellung des Reizerfolges bzw. -mißerfolges sogar früher) kann der Reizerfolg durch Auftreten eines evozierten Potentiales (ventrikulär oder atrial) festgestellt werden. Im Fall einer unterschwelligen Stimulation (Reiz-Mißerfolg) wird also keine Herzaktion tatsächlich ausgesetzt, sondern nur das aktuelle RR-Intervall um 50 ms verlängert.

Ob der zweite Ladekondensator permanent geladen mitgeführt oder nur vor der jeweiligen Inanspruchnahme kurzzeitig aufgeladen wird, hängt davon ab, welche Option bezüglich der gesamten Batteriebeanspruchung (also Leckstrom, aber auch Aufwand für Steuerelektronik usw.) günstiger ist. Grundsätzlich sind beide Varianten möglich. Die Steuereinheit ist in diesem Fall so ausgebildet, dass auch beim zweiten Stimulus alle entsprechenden Maßnahmen wie Blanking usw. wieder vorgenommen werden, d.h. auch der zweite Stimulationsvorgang ist insofern ein normaler Stimulationsvorgang, der jedoch durch die Erfolglosigkeit des ersten Vorganges ausgelöst ist.

Wenn der zweite Ladekondensator permanent aufgeladen mitgeführt wird, und zwar mit einer Spannung, die relativ geringfügig unter der aktuell zum Stimulieren mit dem ersten Ladekondensator verwendeten Spannung liegt, dann ist es auch möglich schnell darauf zu reagieren, wenn die aktuell zum Stimulieren verwendete Spannung durch einen Anstieg der Stimulationsschwelle (wie das typischerweise in den ersten Wochen nach einer Elektrodenimplantation auftritt) unterschwellig wird. Die Feststellung der unterschwelligen Stimulation erfolgt wie vorstehend beschrieben innerhalb von höchstens 50 ms nach dem Nichtauftreten des evozierten Potentiales. In diesem Fall wird der zweite Ladekondensator, der ja ohnehin schon deutlich aufgeladen ist, auf eine Spannung aufgeladen, die über der nun unterschwellig gewordenen, jedoch bisher überschwelligen Ladespannung des ersten Kondensators liegt. Dieser Nachladevorgang kann viel schneller durchgeführt werden als ein vollständiges Wiederaufladen des ersten Kondensators, d.h. wiederum wird kein annähernd vollständiges RR-Intervall, sondern nur Teile davon verloren gehen, bis es zur erfolgreichen Nachstimulation kommt.

Als Kriterien für das Reduzieren der Stimulationsspannung, d.h. z.B. Umschalten auf den zweiten Ladekondensator, kommen mehrere Alternativen in Frage.

Eine dieser Alternativen ist das bereits zuvor genannte Umschalten aufgrund einer Zeitbestimmung, bei welcher mit einer zweiten Elektrode (die vorzugsweise nur zum Sensing verwendet wird) der mit der ersten Elektrode (der eigentlichen Stimulationslektrode) erzielte Reizerfolg erfasst und anschließend analysiert wird. Es ist bekannt, dass eine Reizung nur dann erfolgreich ist, wenn die Reizstärke im erregbaren Gewebe außerhalb der die Reizelektrode umgebenden und mit der Zeit nach Implantation wachsenden fibrotischen Kapsel oberhalb des Schwellenwertes liegt.

Wenn die Reizstärke nun diesen Schwellenwert nicht nur am Rand der fibrotischen Kapsel erreicht, sondern noch deutlich in das erregbare Gewebe außerhalb der fibrotischen Kapsel hineinreicht, dann ist der Weg, den die Erregung bis zur Sensingelektrode zurücklegen muss, kürzer, d.h. diese Erregung wird von der Sensingelektrode früher wahrgenommen als eine Erregung, die dadurch ausgelöst worden ist, dass die Schwellenreizstärke nur gerade über den Rand einer sich um eine implantierte Elektrode herum bildenden fibrotischen Kapsel hinausgereicht hat. Dem liegt die Erkenntnis zugrunde, dass die Ausbreitungsgeschwindigkeit des elektrischen Feldes, ausgehend von der Stimulationselektrode, deutlich größer ist als die Ausbreitungsgeschwindigkeit der Erregung. Die Erregung geht dabei grundsätzlich immer von jener Randzone aus, in der gerade die Schwellenreizstärke erreicht wird. Selbstverständlich kann beim Verwenden einer zweiten Elektrode (als Sensingelektrode) diese auch ganz grundsätzlich zum Nachweis des Reizerfolges bzw. -mißerfolges verwendet werden.

Alternativ oder zusätzlich zu dem vorbeschriebenen Zeitkriterium kann auch ein "blindes" Umschalten nach einer vorgegebenen Anzahl von erfolgreichen Stimulationsvorgängen mit einer ersten, überschwelligen Stimulationsintensität vorgesehen werden. Die Steuereinheit ist dann so ausgebildet, dass nach einer vorgegebenen Zahl erfolgreicher Stimulationen auf eine niedrigere Stimultionsintensität, insbesondere auf den zweiten Ladekondensator umgeschaltet wird. Eine solche Anzahl kann z.B. 1000 sein. Die Steuereinheit umfasst vorzugsweise einen Zähler für die Anzahl aufeinanderfolgender erfolgreicher Stimulationen, der im Falle eines Stimulationsmißerfolges oder beim Umschalten auf eine niedrigere Stimulationsintensität zurückgesetzt wird.

Falls die Stimulationseinheit zwei Ladekondensatoren umfasst, wird beispielsweise nach jedem 1000. erfolgreichen Stimulus auf den zweiten Ladekondensator umgeschaltet und bei erfolgreichem Stimulieren dessen Ladespannung beibehalten, wodurch der zweite Ladekondensator zum ersten wird und der bisherige erste die Rolle des zweiten übernimmt, d.h. jetzt mit einer geringeren Spannung aufgeladen wird als der neue erste Ladekondensator (insofem sind beide Ladekondensator als gleichwertig zu erachten, d. h. wer erster Ladekondensator ist, hängt von den jeweils aktuellen Bedingungen ab). Wiederum nach 1000 erfolgreichen Stimulationen wird eine erneute Absenkung der Stimulationsspannung vorgenommen. Bei nicht erfolgreicher Stimulation wird nach spätestens 50 ms auf den ersten Ladekondensator zurückgeschaltet. Zugleich sollte nach einer nicht erfolgreichen Absenkung der Stimulationsspannung die Zahl von 1000 bis zum nächsten "blinden" Versuch erhöht werden, z.B. auf 5000, um häufiges erfolgloses Stimulieren zu verhindern.

Ein weiteres, auch unabhängig umzusetzendes Kriterium für das Umschalten der Stimulationsintesität ist das Umschalten aufgrund bestimmter Merkmale in der Morphologie der evozierten Potentiale (ventrikulär oder atrial), die darauf schließen lassen, dass die aktuelle Reizstärke (d.h. die Stimulations- bzw. Ladespannung des ersten Ladekondensators) deutlich über der Reizschwelle liegt. Solche Merkmale in der Signalmorphologie können bestimmte Amplitudenwerte sein (z.B. Maximalwerte bzw. Werte, die durch eine deutliche Änderung in der Morphologie bzw. im Signalcharakter identifizierbar sind), Zeitpunkte für das Auftreten bestimmter Amplitudenwerte oder Momente höherer Ordnung, z.B. Steilheiten im Signalverlauf, die durch Differenzieren erhalten werden können, oder Integrale zwischen vorgegebenen Primärmerkmalen (also zwischen Amplitudenwerten bzw. dem Zeitpunkt ihres Auftretens), oder dem Verhältnis von zwei ausgewählten Amplitudenwerten. Die Feststellung solcher Änderungen in der Signalmorphologie sollte sich vorzugsweise auf einen Musterkomplex des betreffenden (individuellen) Signales beziehen, der durch Mittelwertbildung (z. B. über 100 Einzelsignale gewonnen wird, eventuell mit Gewichtung hinsichtlich des Zeitpunktes des Auftretens vor dem jeweils aktuellen Einzelsignal) gewonnen wird. Ob eine im Sinne der Erniedrigung der Ladespannung für den ersten Kondensator hinreichende Abweichung vorliegt, ist vorzugsweise durch einen Schwellenwert vorgegeben. Dieser Schwellenwert kann sich prozentual auf den Mittelwert desselben Signalwertes beziehen, z.B. Änderung um 10%, oder auf die laufend festgestellte Standardabweichung (z.B. beim Überschreiten der einfachen oder doppelten Standardabweichung) oder auf einen Relativwert, z.B. Verhältnis von Standardabweichung zum Mittelwert.

Vorteilhafterweise ist das Stimulationsgerät ausgebildet, die Stimulationsintensität entweder durch Verlängern der Impulsdauer oder Erhöhen der Impulsstärke zu vergrößern oder andererseits die Stimulationsintensität durch Verringern der Stimulationsintensität oder durch Verkürzen der Impulsdauer zu bewirken. Dies kann durch das vorbeschriebene Umschalten zwischen zwei Kondensatoren geschehen.

In einer Ausführungsvariante ist eine gemeinsame Elektrode zur Abgabe des Stimulationsimpulses und zur Aufnahme des aufzunehmenden Signals für die Stimulationserfolgskontrolle vorgesehen. In diesem Fall ist die Elektrode sowohl mit der Stimulationseinheit als auch mit dem Signaldetektor verbunden.

In einer bevorzugten Ausführungsvariante sind zwei separate Elektroden für die Stimulationsimpulsabgabe und die Aufnahme des Signals vorgesehen. Die für die Aufnahme des Signals vorgesehene Abfühlelektrode ist dann mit dem Signaldetektor verbunden, während die für die Abgabe des Stimulationsimpulses vorgesehene Elektrode mit der Stimulationseinheit verbunden ist.

Wie bereits zuvor erläutert, ist das Stimulationsgerät dazu ausgebildet, die Stimulationsintensität zu verringern, falls die Zeitdifferenz zwischen Abgabe des Stimulationsimpulses und Erfassen des Stimulationserfolges kürzer als ein gespeicherter Referenzwert ist.

Zusätzlich zu dieser Variante der Stimulationsintensitätsvariation kann auch ein Morphologiedetektor vorgesehen sein, der mit dem Signaldetektor und der Steuereinheit verbunden und ausgebildet ist, morphologische Merkmale des aufgenommenen Signals zu detektieren und beim Auftreten vorbestimmter morphologischer Merkmale ein Morphologiesignal an die Steuereinheit auszugeben. Dieses Morphologiesignal kann einerseits dazu dienen, das Erkennen bestimmter Signalmerkmale nach Art eines Filters zu ermöglichen, um auf diese Weise die Zeitdifferenz zwischen Abgabe des Stimulationsimpulses und Auftreten des Signalmerkmales zu ermöglichen. Andererseits kann aus der Signalmorphologie selbst ein ggf. zusätzliches Signal für die Variation der Stimulationsintensität abgeleitet werden.

In vorteilhafter Weise ist das Stimulationsgerät dazu ausgebildet, einen zweiten Stimulationsimpuls größerer Stimulationsintensität als Backup-Impuls auszulösen, falls nach Ablauf in einer vorgegebenen Zeit nach Abgabe eines ersten Stimulationsimpulses kein Stimulationserfolg detektiert wird.

In einer besonders bevorzugten Variante ist das Stimulationsgerät ausgebildet, den Referenzwert für die Zeitdifferenz oder auch die vorbestimmten morphologischen Merkmale durch Autokalibration selbständig zu bestimmen. Varianten für vorteilhafte Ausgestaltungen der entsprechenden Autokalibrationseinheit sind den Unteransprüchen zu entnehmen.

Schließlich ist das Stimulationsgerät vorzugsweise als Herzschrittmacher ausgebildet.

Weitere vorteilhafte Ausgestaltungsvarianten sind insbesondere der folgenden Figurenbeschreibung zu entnehmen.

Die Erfindung soll nun anhand von Ausführungsbeispielen mit Hilfe der Figuren näher erläutert werden. Von den Figuren zeigen:
- Figur 1a und b: eine erste Variante eines Stimulationsgerätes mit zeitdifferenzgesteuerter Stimulationsintensität; in zwei Untervarianten
- Figur 2: eine zweite Ausführungsvariante des Stimulationsgerätes mit signal-morphologiegesteuerter Stimulationsintensität.

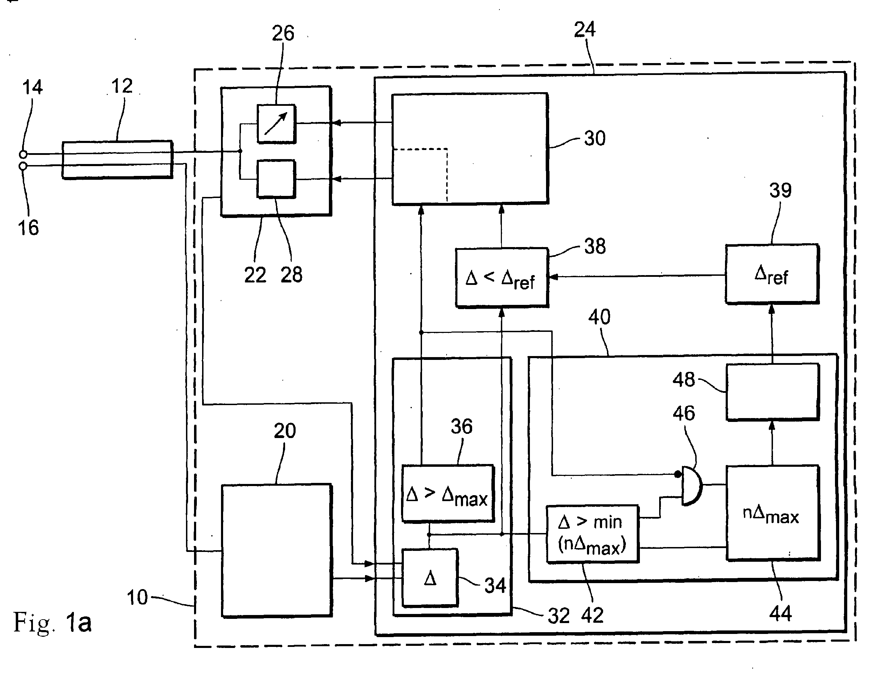

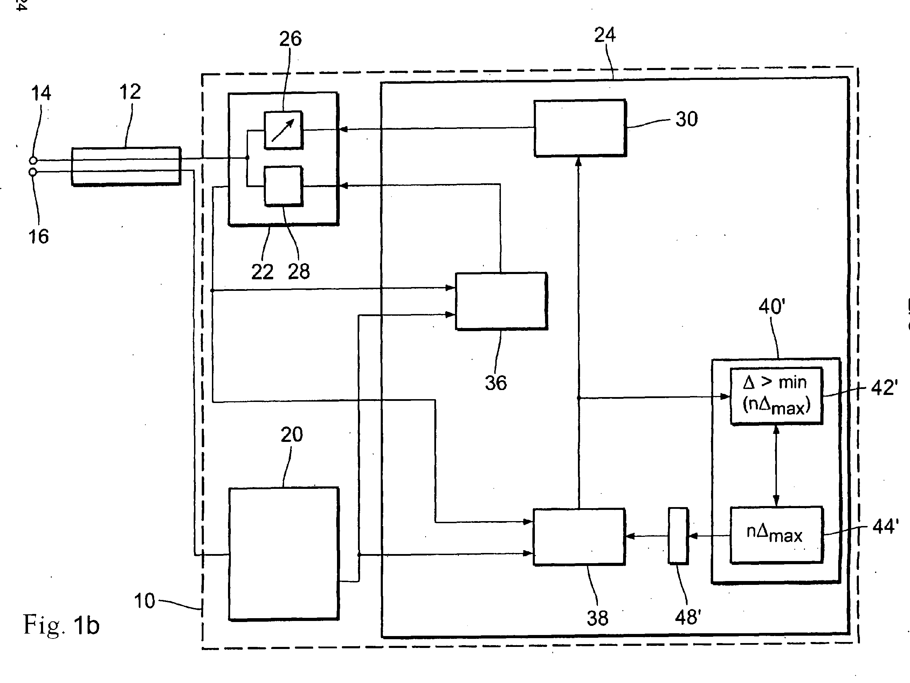

Figur 1 zeigt ein als Herzschrittmacher 10 ausgebildetes Stimulationsgerät. Der Herzschrittmacher 10 ist über einen Elektrodenkatheter 12 mit einer Stimulationselektrode 14 und einer Abfühlelektrode 16 verbunden.

Der Herzschrittmacher 10 umfasst eine Stimulationseinheit 22, einen Signaldetektor 20 und eine Steuereinheit 24.

Der Signaldetektor 20 ist eingangsseitig mit der Abfühlelektrode 16 verbunden. Der Signaldetektor 20 ist ausgebildet, ausgangsseitig ein Signal auszugeben, wenn über die Abfühlelektrode 16 ein Signal aufgenommen wird, insbesondere, wenn der Signaldetektor 20 ein bestimmtes Merkmal in einem von der Abfühlelektrode 16 aufgenommenen Signal erfasst. Dieses Merkmal kann beispielsweise die erste ansteigende Signalflanke des aufgenommenen Signals sein. Auf die letztgenannte Art und Weise wird eine schnellstmögliche Signaldetektion ermöglicht.

Die Stimulationseinheit 22 ist ausgangsseitig zum einen mit der Stimulationselektrode 14 verbunden. Die Stimulationseinheit 22 umfasst zwei Impulsgeber, einen steuerbaren Impulsgeber 26 und einen Backup-Impulsgeber 28. Beide Impulsgeber 26 und 28 sind mit der Stimulationselektrode 14 verbunden und ausgebildet, einen elektrischen Stimulationsimpuls an die Stimulationselektrode 14 abzugeben.

Der steuerbare Impulsgeber 26 umfasst einen Kondensator, dessen Ladung in Abhängigkeit eines variierbaren Steuersignals variiert werden kann. Konkret wird der Kondensator des steuerbaren Impulsgebers 26 auf eine durch das variierbare Steuersignal vorzugebenen Spannung aufgeladen. Der Backup-Impulsgeber 28 umfasst einen Kondensator der immer auf einen festen Spannungswert aufgeladen wird. Der steuerbare Impulsgeber 26 ist in der Lage, einen hinsichtlich der Stimulationsintensität variierbaren Stimulationsimpuls abzugeben. Eine Variation des Stimulationsimpulses hinsichtlich der Stimulationsstärke ist über Variation der Ladespannung des Kondensators des steuerbaren Impulsgebers 26 möglich. Darüber hinaus kann die Stimulationsintensität auch zusätzlich oder alternativ dadurch variiert werden, dass die Impulsdauer des von dem steuerbaren Impulsgeber 26 ausgegebenen Stimulationsimpulses variiert wird. Ein entsprechender Impulsdauertimer ist dann über ein variierbares Steuersignal entsprechend einstellbar ausgestaltet. Die Stimulationsintensität des von dem steuerbaren Impulsgeber 26 abgegebenen Stimulationsimpulses wird jeweils gerade so eingestellt, dass die Stimulationsintensität ausreicht, das Herz, insbesondere den Ventrikel oder das Atrium eines Patienten gleichzeitig mit möglichst minimalem Energieaufwand und möglichst sicherem Stimulationserfolg zu stimulieren. Die Steuereinheit 24 ist daher dazu ausgebildet, regelmäßig den steuerbaren Impulsgeber 26 anzusteuern.

Nur falls mittels der Steuereinheit 24 in Verbindung mit dem Signaldetektor 20 kein Stimulationserfolg nach Abgabe eines Stimulationsimpulses über den steuerbaren Impulsgeber 26 erfasst wird, löst die Steuereinheit 24 den Backup-lmpulsgeber 28 aus. Dieser ist so ausgelegt, dass der von dem Backup-Impulsgeber 28 abgegebene Stimulationsimpuls eine Stimulationsintensität hat, die mit Sicherheit über der Reizschwelle des zu stimulierenden Herzgewebes liegt. Der Kondensator des Backup-Impulsgebers 28 wird daher regelmäßig entsprechend hoch geladen.

Zum Ansteuern der Stimulationseinheit 22 umfasst die Steuereinheit 24 eine Stimulationsansteuereinheit 30, die ausgangsseitig zum Ansteuern der Stimulationseinheit 22 sowohl mit dem steuerbaren Impulsgeber 26 als auch mit dem Backup-Impulsgeber 28 verbunden ist.

Außerdem umfasst die Steuereinheit 24 eine Zeitbestimmungseinheit 32, die eingangsseitig sowohl mit dem Signaldetektor 20 als auch mit der Stimulationseinheit 22 verbunden ist. Auf diese Weise empfängt die Zeitbestimmungseinheit 32 von der Stimulationseinheit 22 ein Signal, sobald die Stimulationseinheit 22 einen Stimulationsimpuls abgibt. Von dem Signaldetektor 20 empfängt die Zeitbestimmungseinheit 32 ein Signal, wenn der Signaldetektor 20 ein aufgenommenes Signal oder ein Merkmal eines aufgenommenen Signals detektiert.

Über einen Differenzwertbildner 34 der Zeitbestimmungseinheit 32 wird die Zeitdifferenz zwischen der Abgabe eines Stimulationsimpulses und dem Auftreten eines aufgenommenen Signals oder eines Signalmerkmales bestimmt. Ein dieser Zeitdifferenz entsprechendes Signal wird an einen Differenzwerttimer 36 der Zeitbestimmungseinheit 32 ausgegeben. In dem Differenzwerttimer 36 wird bestimmt, ob die Zeitdifferenz zwischen Stimulationsimpuls und erfasstem Signalmerkmal über einem vorgegebenen Maximalwert liegt. Falls dieser Maximalwert überschritten wird, wird unverzüglich ein Signal an die Stimulationsansteuereinheit 30 ausgegeben, die daraufhin unmittelbar den Backup-Impulsgeber 28 ansteuert. Auf diese Weise ist sichergestellt, dass ein Backup-Stimulationsimpuls ausgegeben wird, falls ein zuvor abgegebener gesteuerter Stimulationsimpuls nicht zu einem Stimulationserfolg geführt hat.

Anstelle der in Figur 1a dargestellten Ausführungsvariante, bei der die Signale von der Stimulationseinheit 22 und vom Signaldetektor 20 dem Differenzwertbildner 34 zugeführt werden, und dann erst dem Differenzwerttimer 36, kann der Differenzwerttimer 36 auch unmittelbar mit der Stimulationseinheit 22 und dem Signaldetektor 20 verbunden sein und tatsächlich als Timer ausgestaltet sein. In diesem in Figur 1b dargestellten Fall wird der Differenzwerttimer durch einen von der Stimulationseinheit 22 gleichzeitig mit Abgabe eines Stimulationsimpulses ausgegebenes Signal gestartet und durch ein von dem Signaldetektor 20 stammendes Signal zurückgesetzt. Falls der Differenzwerttimer 36' nicht vor Ablauf einer vorgegebenen Zeit durch ein von dem Signaldetektor 20 stammendes Signal zurückgesetzt wird, gibt der Differenzwerttimer 36' mit Ablauf der vorgegebenen Zeit unmittelbar ein Signal ab, das zum Auslösen des Backup-Impulsgebers 28 führt. Der Differenzwerttimer 36' kann dazu ausgangsseitig auch direkt mit dem Backup-Impulsgeber 28 verbunden sein.

Zur Steuerung der Stimulationsintensität regulärer, gesteuerter Stimulationsimpulse, die durch den steuerbaren Impulsgeber 26 abgegeben werden, umfasst die Steuereinheit 24 eine Referenzzeitdifferenzeinheit 38, welche in einer Ausführungsvariante (Figur 1a) mit dem Differenzwertbildner 34 verbunden ist und ein der Zeitdifferenz zwischen Abgabe eines Stimulationsimpulses durch die Stimulationseinheit 22 und Aufnahme eines Signalmerkmales durch den Signaldetektor 20 entsprechendes Zeitsignal empfängt. In der Referenzzeitdifferenzeinheit 38 wird diese Zeitdifferenz mit einem Referenzwert verglichen und ein zur Verringerung der Stimulationsintensität dienendes Signal an die Stimulationsansteuereinheit 30 abgegeben, falls die Zeitdifferenz kleiner als der Referenzwert ist, oder die Zeitdifferenz um einen vorgegebenen Mindestbetrag kleiner als der Referenzwert ist. Der Referenzwert ist in einem Referenzwertspeicher 39 gespeichert.

In einer alternativen Ausführungsvariante (Figur 1b) kann die Referenzzeitdifferenzeinheit 38' auch als Timer ausgebildet sein, der eingangsseitig unmittelbar mit der Stimulationseinheit 22 zur Aufnahme eines Signals bei Abgabe eines Stimulationsimpulses und mit dem Signaldetektor 20 zur Abgabe eines Signals bei Erfassen vorgegebener Signalmerkmale verbunden sein. Die als Timer ausgebildete Referenzzeitdifferenzeinheit 38 wird in dieser Ausführungsvariante genau wie der Differenzwerttimer 36' durch ein Signal der Stimulationseinheit 22 gestartet und durch ein Signal von dem Signaldetektor 20 zurückgesetzt. Der Unterschied zwischen der Referenzzeitdifferenzeinheit 38' und dem Differenzwerttimer 36' besteht in diesem Fall zum einen darin, dass die durch den Referenzwert vorgegebene Laufzeit der Referenzzeit der Referenzzeitdifferenzeinheit 38 variabel ist, während der Differenzwerttimer 36' nach fest vorgegebener Laufzeit abläuft. Außerdem ist die Laufzeit des Differenzwerttimers 36' größer, als die Laufzeit der Referenzzeitdifferenzeinheit 38'.

Die Referenzzeitdifferenzeinheit 38 gibt ein der Abweichung der Zeitdifferenz zwischen Abgabe eines Stimulationsimpulses und Detektion eines Stimulationserfolges von dem Referenzwert entsprechendes Signal aus, welches über die Stimulationsansteuereinheit 30 die Variation des steuerbaren Impulsgebers 26 bewirkt. Die Abweichung der Zeitdifferenz zwischen Stimulation und Stimulationserfolg vom Referenzwert ergibt sich bei der Variante, bei der die Referenzzeit der Referenzzeitdifferenzeinheit 38 als Timer ausgebildet ist, aus der Restlaufzeit dieses Timers zwischen dem Zurücksetzen des Timers durch den Signaldetektor 20 und Ablaufen des Timers nach der durch den Referenzwert vorgegebenen Zeit.

Wird der Timer der Referenzzeitdifferenzeinheit 38 zurückgesetzt, bevor er nach der durch den Referenzwert vorgegebenen Zeit abläuft, wird der steuerbare Impulsgeber 26 so angesteuert, dass die Stimulationsintensität des nächsten Stimulationsimpulses um einen der Abweichung zwischen Zeitdifferenz und Referenzwert entsprechenden Wert verringert. Falls die Zeitdifferenz nicht geringer ist als der Referenzwert, falls also der Timer der Referenzzeitdifferenzeinheit 38 nicht vor Ablauf nach durch den Referenzwert vergebener Zeit durch ein Signal des Signaldetektors 20 zurückgesetzt wird, erfolgt keine Änderung des steuerbaren Impulsgebers 26 im Hinblick auf eine veränderte Stimulationsintensität. Es kann sogar vorgesehen sein, im letztgenannten Fall die Stimulationsintensität zu erhöhen.

Die Änderung der Stimulationsintensität kann wie zuvor beschrieben durch Variieren der Impulsdauer, also Einstellen eines entsprechenden Timers der steuerbaren Impulsgeber 26, oder Variieren der Stimulationsstärke, also Variieren der Ladespannung des Kondensators des steuerbaren Impulsgebers 26, oder durch beide Maßnahmen erfolgen.

Die Steuereinheit 24 ist mit einer Autokalibrationseinheit 40 versehen, über die die Steuereinheit 24 in der Lage ist, den in dem Referenzwertspeicher selbstständig zu bestimmen. Die Autokalibrationseinheit 40 ist ausgebildet, einen geeigneten Referenzwert zu bilden. In der in Figur 1a dargestellten Ausführungsvariante umfasst die Autokalibrationseinheit 40 dazu eine Differenzwertvergleichseinheit 42, die eingangsseitig einerseits mit dem Differenzwertbildner 34 und andererseits mit einem Differenzwertspeicher 44 verbunden ist. In dem Differenzwertspeicher 44 ist eine vorgegebene Anzahl der längsten jeweils ermittelten Zeitdifferenzen gespeichert. Die Differenzwertvergleichseinheit vergleicht eine jeweils aktuelle Zeitdifferenz mit einer der in dem Differenzwertspeicher 44 gespeicherten Zeitdifferenzen und bestimmt, ob die jeweils aktuelle Zeitdifferenz größer ist, als die kleinste der in dem Differenzwertspeicher 44 gespeicherten Zeitdifferenzen. Falls dies so ist, wird die kürzeste der in dem Differenzwertspeicher 44 gespeicherten Zeitdifferenzen durch die jeweils aktuelle Zeitdifferenz ersetzt. Dieses Speichern der jeweils aktuellen Zeitdifferenz wird nur dann durch ein UND-Glied 46 unterbunden, wenn die aktuelle Zeitdifferenz größer ist, als der für den Differenzwerttimer 36 vorgegebene Maximalwert, falls also der Differenzwerttimer 36 ein Ausgangssignal abgibt. Dieses Ausgangssignal wird auf einen invertierten Eingang des UND-Gatters 46 gegeben und verhindert somit das Speichern der jeweils aktuellen Zeitdifferenz. Die Autokalibrationseinheit 40 umfasst weiterhin einen Mittelwertbildner 48, der mit dem Differenzwertspeicher 44 verbunden ist und den Mittelwert der in dem Referenzwertspeicher 44 gespeicherten Zeitdifferenzen bildet, sobald die in dem Differenzwertspeicher 44 gespeicherten Zeitdifferenzen verändert werden. Ausgangsseitig gibt der Mittelwertbildner den Mittelwert der in dem Differenzwertspeicher 44 gespeicherten Zeitdifferenzen als Referenzwert in den Referenzwertspeicher 39. Der Referenzwertspeicher 39 ist - wie zuvor beschrieben - mit der Referenzzeitdifferenzeinheit 38 verbunden.

In der in Figur 1b abgebildeten Alternative ist die Referenzzeitdifferenzeinheit 38 als Timer ausgebildet und bestimmt - wie zuvor beschrieben - die zwischen Reset des Timers und Laufzeit des Timers verbleibende Restzeit oder die nach Ablaufen der durch den Referenzwert bestimmten Zeit bis zum Eintreffen des Signals zum Signaldetektor 20 verstreichende Zeit. Auf diese Weise liegt am Ausgang der Referenzzeitdifferenzeinheit 38 ein vorzeichenbehaftetes Zeitsignal an, welches in einer Differenzwertvergleichseinheit 42' mit einer Anzahl von in einem Differenzwertspeicher 44' gespeicherten Differenzwerten verglichen wird. Unter Berücksichtigung des Vorzeichens des derart bestimmten Zeitsignals wird bestimmt, ob das aktuelle Zeitdifferenzsignal größer ist, als der kleinste Zeitdifferenzsignalwert in dem Differenzwertspeicher 44' und gegebenenfalls der kleinste Zeitdifferenzsignalwert durch den aktuellen Zeitdifferenzsignalwert ersetzt. Ein mit dem Differenzwertspeicher 44' verbundener Mittelwertbildner 48' addiert bei Veränderung der in dem Differenzwertspeicher 44' gespeicherten Werte diese Werte jeweils mit dem Referenzwert, der gleichzeitig die Laufzeit der als Timer ausgebildeten Referenzzeitdifferenzeinheit 38 ist. Auf diese Weise ergeben sich ebensoviele Zeitwerte, wie Zeitsignaldifferenzwerte in dem Referenzwertspeicher 44' gespeichert sind. Diese Zeitdifferenzen werden gemittelt und ergeben den jeweilig aktuellen Referenzwert.

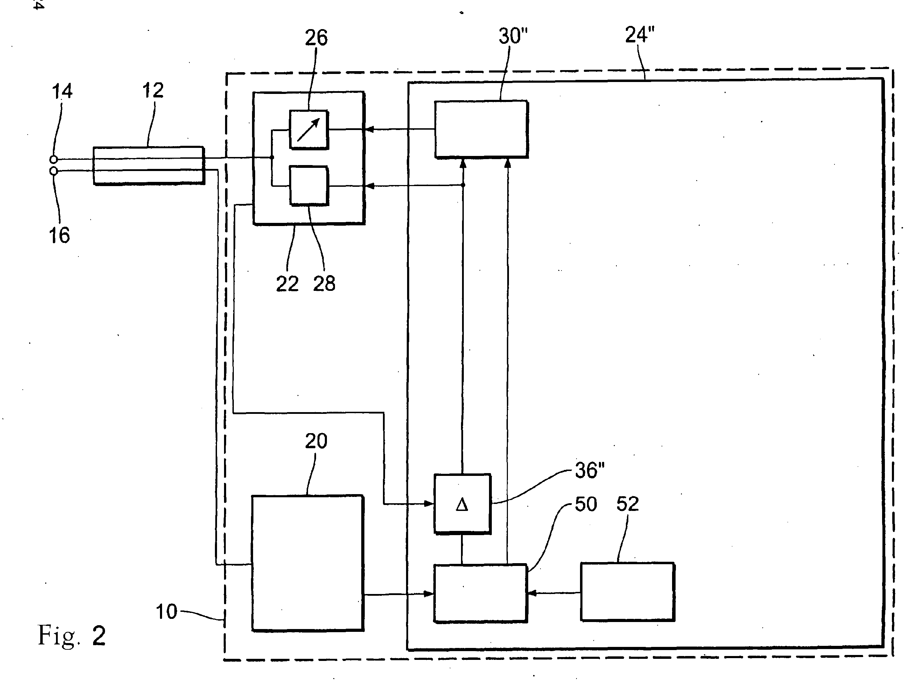

Figur 2 zeigt einen alternativen Herzschrittmacher 10. Dessen Elektrodenkatheder 12 mit Stimulationselektrode 14 und Abfühlelektrode 16, sowie dessen Signaldetektor 20, und dessen Stimulationseinheit 22 mit einem steuerbaren Impulsgeber 26 und einem Backup-Impulsgeber 28 sind gleich oder ähnlich aufgebaut, wie bei den Herzschrittmachern aus den Figuren 1a und 1b.

Eine alternative Steuereinheit 24" umfasst insbesondere eine Signalmustervergleichseinheit 50, die einerseits mit dem Signaldetektor 20 verbunden ist und andererseits mit einem Signalmusterspeicher 52. Die Signalmustervergleichseinheit 50 ist ausgebildet, von dem Signaldetektor 20 stammende Signalabschnitte eines aufgenommenen Signals mit Signalmustern zu vergleichen, die in dem Signalmusterspeicher 52 gespeichert sind.

Durch diesen Mustervergleich bildet die Signalmustervergleichseinheit 50 einerseits ein Zeitsignal, welches über eine Steuerleitung an einen Differenzwerttimer 36" abgibt. Der Differenzwerttimer 36" ist wie bei der Ausführungsvariante gemäß Figur 1b mit der Stimulationseinheit 22 verbunden und empfängt von dieser ein den Zeitpunkt der Abgabe eines Stimulationsimpulses charakterisierendes Stimulationszeitsignal. In dem Differenzwerttimer 36" wird ein Differenzzeitsignal gebildet, welches von der Zeitdifferenz zwischen der Abgabe eines Stimulationsimpulses über die Stimulationseinheit 22 und dem Detektieren eines Signalmerkmals durch die Signalmustervergleichseinheit 50. Der letztgenannte Zeitpunkt ist durch das Zeitsignal bestimmt, welches die Signalmustervergleichseinheit 50 an den Differenzwerttimer 36" abgibt. Das Differenzzeitsignal wird von dem Differenzwerttimer 36" über eine Signalleitung an die Stimulationsansteuereinheit 30 ausgegeben.

Zusätzlich ist die Signalmustervergleichseinheit 50 ausgebildet, den von der Detektionseinheit 20 stammenden Signalabschnitt mit verschiedenen Vergleichsmustern aus dem Signalmusterspeicher 52 zu vergleichen und dem jeweils Ähnlichsten der Vergleichsmuster zuzuordnen. Je nachdem welchem Vergleichsmuster der aufgenommene Signalabschnitt zugeordnet wird, bildet die Signalmustervergleichseinheit 50 ein Zuordnungssignal, welches ebenfalls an die Stimulationsansteuereinheit 30 ausgegeben wird. Die Stimulationsansteuereinheit 30 bildet aus dem Differenzzeitsignal von dem Differenzwerttimer 36" sowie dem für ein typisches Signalmuster charakteristischen Zuordnungssignal von der Signalmustervergleichseinheit 50 ein Signal zum Ansteuern des steuerbaren Impulsgeber 26. Die Stimulationsintensität eines Stimulationsimpulses, der von dem steuerbaren Impulsgeber 26 abgegeben wird, hängt bei dem Herzschrittmacher gemäß Figur 2 somit sowohl von der Zeitdifferenz zwischen der Abgabe eines Stimulationsimpulses und der Aufnahme eines bestimmten Signalmerkmals ab, als auch von der charakteristischen Form dieses Signalmerkmals.

Daneben wird das reine Zeitdifferenzsignal von dem Differenzwerttimer 36" direkt der Backup-Stimulationseinheit 38 zugeführt, die einen Backup-Stimulationsimpuls der vorbeschriebenen Art auslöst, falls die Zeitdifferenz zwischen der Abgabe eines Stimulationsimpulses und dem Detektieren eines entsprechenden Signalmerkmales einen vorgegebenen Grenzwert überschreitet.

## Patentansprüche

1. Stimulationsgerät, insbesondere für ein menschliches Herz;
- mit einer Stimulationseinheit, die zum Abgeben eines Stimulationsimpulses mit einer Impulsdauer, einer Impulsstärke und einer Stimulationsintensität ausgebildet ist und einen steuerbaren Impulsgeber und einen Backup-Impulsgeber umfasst,
- mit einem Signaldetektor, der zum Detektieren eines Stimulationserfolges anhand eines aufgenommenen Signals ausgebildet ist, und
- mit einer Steuereinheit, die mit der Stimulationseinheit und mit dem Signaldetektor derart verbundenen und ausgebildet ist, dass die Stimulationsintensität in Abhängigkeit des aufgenommenen Signals veränderbar ist, und die zum Ansteuern der Stimulationseinheit eine Stimulationsansteuereinheit umfasst, die ausgangsseitig zum Ansteuern der Stimulationseinheit sowohl mit dem steuerbaren Impulsgeber als auch mit dem Backup-Impulsgeber verbunden ist.
- mit einer Zeitbestimmungseinheit, die zumindest mittelbar mit der Steuereinheit verbunden und mit der eine Zeitdifferenz zwischen der Abgabe eines Stimulationsimpulses und der Aufnahme des aufgenommenen Signals oder eines Signalmerkmals des aufgenommenen Signals erfassbar ist,
wobei die steuereinheit eine Referenzzeitdifferenzeinheit umfasst, die ausgebildet ist, die Zeitdifferenz zwischen Abgabe eines Stimulationsimpulses durch die Stimulationseinheit und Aufnahme eines einen dem Stimulationsimpuls zuzuordnenden Stimulationserfolg kennzeichnenden Signalmerkmales durch den Signaldetektor mit einem Referenzwert zu vergleichen und ein zur Verringerun der Stimulationsintensität um einen einer Abweichung zwischen Zeitdifferenz und Referenzwert entsprechenden Wert dienendes Signal an die Stimulationsansteuereinheit abzugeben, falls die Zeitdifferenz kleiner als der Referenzwert ist, oder die Zeitdifferenz um einen vorgegebenen Mindestbetrag kleiner als der Referenzwert ist.

2. Stimulationsgerät nach Anspruch 1, **dadurch gekennzeichnet, dass** die Steuereinheit oder die Stimulationseinheit ausgebildet sind, eine Vergrößerung der Stimülationsintensität durch Verlängern der Impulsdauer oder Erhöhen der Impulsstärke und eine Verringerung der Stimulationsintensität durch Verkürzen der Impulsdauer oder Emiedrigen der Impulsstärke zu bewirken.

3. Stimulationsgerät nach Anspruch 1, **gekennzeichnet durch** eine Elektrode, die mit der Stimulationseinheit und dem Signaldetektor verbunden ist, und zur Abgabe des Stimulationsimpulses und zur Aufnahme des Signals ausgebildet ist.

4. Stimulationsgerät nach Anspruch 1, **gekennzeichnet durch** zwei Elektroden, die einen Abstand voneinander haben und von denen eine mit der Stimulationseinheit und die andere mit dem Signaldetektor verbunden ist.

5. Stimulationsgerät nach Anspruch 1, **gekennzeichnet durch** einen Morphologiedetektor, der mit dem Signaldetektor und der Steuereinheit verbunden ist und ausgebildet ist, morphologische Merkmale des aufgenommenen Signals zu detektieren und beim Auftreten vorbestimmter morphologischer Merkmale ein Morphologiesignal an die Steuereinheit auszugeben.

6. Stimulationsgerät nach Anspruch 5, **dadurch gekennzeichnet, dass** der Morphologiedetektor einen Signalformspeicher und eine Morphologie Vergleichseinheit umfasst, die ausgebildet ist morphologische Merkmale des aufgenommenen Signals durch Vergleich des aufgenommenen Signals mit Vergleichsignalen aus dem Signalformspeicher zu detektieren.

7. Stimulationsgerät nach Anspruch 5, **dadurch gekennzeichnet, dass** die Steuereinheit ausgebildet ist, eine Änderung, vorzugsweise eine Verringerung der Stimulationsintensität in Abhängigkeit des Morphologiesignals zu bewirken.

8. Stimulationsgerät nach Anspruch 1, **dadurch gekennzeichnet, dass** die Steuereinheit ausgebildet ist, nach Abgeben eines Stimulationsimpulses durch die Stimulationseinheit das Abgeben eines zweiten Stimulationsimpulses größerer Stimulationsintensität auszulösen, falls innerhalb eines vorgegebenen Zeitraums nach Abgabe des ersten Stimulationsimpulses kein aufgenommenes Signal oder kein Signalmerkmal des aufgenommenen Signals durch den Signaldetektor erfasst wird.

9. Stimulationsgerät nach Anspruch 8. **dadurch gekennzeichnet, dass** die Stimulationseinheit für den ersten und den zweiten Stimulationsimpuls jeweils einen Kondensator umfasst.

10. Stimulationsgerät nach Anspruch 5, **dadurch gekennzeichnet, dass** die Steuereinheit zum selbständigen Ermitteln des Referenzwertes oder der vorbestimmten morphologischen Merkmale durch Autokalibration ausgebildet ist.

11. Stimulationsgerät nach Anspruch 10, **dadurch gekennzeichnet, dass** die Autokalibration durch Aufnahme und Vergleichen einer Mehrzahl von Zeitdifferenzen **dadurch** erfolgt, dass die größte Zeitdifferenz oder der Mittelwert der längsten Zeitdifferenzen aus der Mehrzahl der aufgenommenen Zeitdifferenzen zum Referenzwert bestimmt wird.

12. Stimulationsgerät nach Anspruch 10, **dadurch gekennzeichnet, dass** die Autokalibration durch Bestimmen derjenigen Zeitdifferenz erfolgt, die einem Stimulationsimpuls mit der niedrigsten Stimulationsintensität einer Mehrzahl von Stimulationsimpulsen zugeordnet ist.

13. Stimulationsgerät nach Anspruch 10, **dadurch gekennzeichnet, dass** die Autokalibration durch Bestimmen des Mittelwertes derjenigen Zeitdifferenzen erfolgt, die einer Anzahl von Stimulationsimpulsen mit den niedrigsten Stimulationsintensitäten aus einer Mehrzahl von Stimulationsimpulsen zugeordnet sind.

14. Stimulationsgerät nach Anspruch 10, **dadurch gekennzeichnet, dass** die Autokalibration durch Bestimmen derjenigen morphologischen Merkmale erfolgt; die einem Stimulationsimpuls mit der niedrigsten Stimulationsintensität einer Mehrzahl von Stimulationsimpulsen zugeordnet sind.

15. Stimutationsgerät nach Anspruch 10, **dadurch gekennzeichnet, dass** die Autokalibration durch Bestimmen von Mittelwerten derjenigen morphologischen Merkmale erfolgt, die einer Anzahl von Stimulationsimpulsen mit den niedrigsten Stimulationsintensitäten aus einer Mehrzahl von Stimulationsimpulsen zugeordnet sind.

16. Stimulätionsgerät nach Anspruch 1, **dadurch gekennzeichnet, dass** das Stimulationsgerät ein Herzschrittmacher ist.

## Claims

1. A stimulation device, in particular for a human heart,
- with a stimulation unit, which is designed for delivery of a stimulation pulse having a pulse period, a pulse intensity and a stimulation intensity and comprises a controllable pulse generator and a backup pulse generator,
- with a signal detector, which is designed for detecting a capture on the basis of a signal received, and
- with a control unit, which is designed and connected to the stimulation unit and to the signal detector, such that the stimulation intensity is variable as a function of the signal received and comprising a stimulation control unit for controlling the stimulation unit, connected at the output to the controllable pulse generator and to the backup pulse generator for controlling the stimulation unit,
- with a time determination unit, which is connected at least indirectly to the control unit and with which a time difference between the delivery of a stimulation pulse and picking up the signal received or a signal feature of the signal received can be detected,
wherein the control unit comprises a reference time difference unit which is designed to compare the time difference between delivery of a stimulation pulse by the stimulation unit and receipt of a signal feature by the signal detector, said signal characterizing a capture to be assigned to the stimulation pulse to compare it with a reference value and to deliver a signal, which serves to reduce the stimulation intensity by a value corresponding to a deviation between the time difference and the reference value, to the stimulation control unit if the time difference is smaller than the reference value or if the time difference is smaller than the reference value by a predefined minimum amount.

2. The stimulation device according to claim 1,
**characterized in that**
the control unit or the stimulation unit is designed to induce an increase in the stimulation intensity by prolonging the pulse period or by increasing the pulse intensity and reducing the stimulation intensity by shortening the pulse period or reducing the pulse intensity.

3. The stimulation device according to claim 1,
**characterized by**
an electrode, which is connected to the stimulation unit and the signal detector and is designed for delivery of the stimulation pulse and for receiving the signal.

4. The stimulation device according to claim 1,
**characterized by**
two electrodes spaced a distance apart, one of which is connected to the stimulation unit and the other is connected to the signal detector.

5. The stimulation device according to claim 1,
**characterized by**
a morphology detector, which is connected to the signal detector and the control unit and is designed to detect morphological features of the signal received and to output a morphology signal to the control unit when predetermined morphological features occur.

6. The stimulation device according to claim 5,
**characterized in that**
the morphology detector comprises a signal shape memory and a morphology comparator, which is designed to detect morphological signals of the signal received by comparison of the received signal with reference signals from the signal shape memory.

7. The stimulation device according to claim 5,
**characterized in that**
the control unit is designed to induce a change, preferably a change in the stimulation intensity as a function of the morphology signal.

8. The stimulation device according to claim 1,
**characterized in that**
the control unit is designed to trigger the delivery of a second stimulation pulse of a greater stimulation intensity after delivery of a stimulation pulse by the stimulation unit, if no received signal or no signal feature of the signal received is detected by the signal detector within a predefined period of time after the delivery of the first stimulation pulse.

9. The stimulation device according to claim 8,
**characterized in that**
the stimulation unit comprises one capacitor each for the first stimulation pulse and for the second stimulation pulse.

10. The stimulation device according to claim 5,
**characterized in that**
the control unit is designed for automatically determining the reference value or the predetermined morphological features by autocalibration.

11. The stimulation device according to claim 10,
**characterized in that**
the autocalibration is performed by receiving and comparing a plurality of time differences by determining the greatest time difference or the average of the longest time differences from the plurality of received time differences to the reference value.

12. The stimulation device according to claim 10,
**characterized in that**
the autocalibration is performed by determining the time difference, which is assigned to a stimulation pulse with the lowest stimulation intensity of a plurality of stimulation pulses.

13. The stimulation device according to claim 10,
**characterized in that**
the autocalibration is performed by determining the average of the time differences, which are assigned to a number of stimulation pulses having the lowest stimulation intensities from a plurality of stimulation pulses.

14. The stimulation device according to claim 10,
**characterized in that**
the autocalibration is performed by determining the morphological features, which are assigned to a stimulation pulse having the lowest stimulation intensity of a plurality of stimulation pulses.

15. The stimulation device according to claim 10,
**characterized in that**
the autocalibration is performed by determining averages of the morphological features, which are assigned to a number of stimulation pulses having the lowest stimulation intensities from a plurality of stimulation pulses.

16. The stimulation device according to claim 1,
**characterized in that**
the stimulation device is a cardiac pacemaker.

## Revendications

1. Appareil de stimulation, en particulier pour un coeur humain, comprenant
- une unité de stimulation qui est conçue pour la délivrance d'une impulsion de stimulation avec une durée d'impulsion, une intensité d'impulsion et une intensité de stimulation et comporte un générateur d'impulsions contrôlable et un générateur d'impulsions de sauvegarde
- un détecteur de signal, qui est conçu pour la détection d'un résultat de stimulation à l'aide d'un signal enregistré et
- une unité de commande, qui est reliée à l'unité de stimulation et au détecteur de signal et est conçue de telle sorte que l'intensité de stimulation est variable en fonction du signal enregistré, et qui comporte pour l'activation de l'unité de stimulation une unité d'activation de stimulation qui est reliée côté sortie pour l'activation de l'unité de stimulation aussi bien au générateur d'impulsions contrôlable qu'au générateur d'impulsions de sauvegarde,
- une unité de détermination de temps, qui est reliée au moins indirectement à l'unité de commande et avec laquelle une différence de temps entre la délivrance d'une impulsion de stimulation et l'enregistrement du signal enregistré ou d'une caractéristique de signal du signal enregistré peut être saisie,
- l'unité de commande comprenant une unité de différence de temps de référence qui est conçue pour comparer la différence de temps entre la délivrance d'une impulsion de stimulation par l'unité de stimulation et l'enregistrement d'une caractéristique de signal caractérisant un résultat de stimulation à attribuer à l'impulsion de stimulation par le détecteur de signal avec une valeur de référence et pour délivrer un signal servant à réduire l'intensité de stimulation d'une valeur correspondant à un écart entre la différence de temps et la valeur de référence à l'unité d'activation de stimulation dans le cas où la différence de temps est inférieure à la valeur de référence ou dans le cas où la différence de temps est inférieure d'une valeur minimale prédéfinie à la valeur de référence.

2. Appareil de stimulation selon la revendication 1, **caractérisé en que** l'unité de commande ou l'unité de stimulation est conçue pour entraîner une augmentation de l'intensité de stimulation par le prolongement de la durée d'impulsion ou l'élévation de l'intensité d'impulsion et une réduction de l'intensité de stimulation par le raccourcissement de la durée d'impulsion ou l'abaissement de l'intensité d'impulsion.

3. Appareil de stimulation selon la revendication 1, **caractérisé par** une électrode, qui est reliée à l'unité de stimulation et au détecteur de signal, et est conçue pour la délivrance de l'impulsion de stimulation et pour l'enregistrement du signal.

4. Appareil de stimulation selon la revendication 1, **caractérisé par** deux électrodes, qui sont espacées l'une de l'autre et dont l'une est reliée à l'unité de stimulation et l'autre au détecteur de signal.

5. Appareil de stimulation selon la revendication 1, **caractérisé par** un détecteur de morphologie, qui est relié au détecteur de signal et à l'unité de commande et est conçu pour détecter des caractéristiques morphologiques du signal enregistré et envoyer un signal de morphologie à l'unité de commande dans le cas d'apparition de caractéristiques morphologiques prédéfinies.

6. Appareil de stimulation selon la revendication 5, **caractérisé en ce que** le détecteur de morphologie comporte une mémoire de forme de signal et une unité de comparaison de morphologie qui est conçue pour détecter des caractéristiques morphologiques du signal enregistré par comparaison du signal enregistré avec des signaux de comparaison provenant de la mémoire de forme de signal.

7. Appareil de stimulation selon la revendication 5, **caractérisé en que** l'unité de commande est conçue pour entraîner une modification, de préférence une réduction de l'intensité de stimulation en fonction du signal de morphologie.

8. Appareil de stimulation selon la revendication 1, **caractérisé en que** l'unité de commande est conçue pour déclencher, après la délivrance d'une impulsion de stimulation par l'unité de stimulation, la délivrance d'une seconde impulsion de stimulation d'intensité de stimulation plus grande dans le cas où, dans une période prédéfinie et après la délivrance de la première impulsion de stimulation, aucun signal enregistré ou aucune caractéristique de signal du signal enregistré n'est détecté par le détecteur de signal.

9. Appareil de stimulation selon la revendication 8, **caractérisé en que** l'unité de stimulation comporte à chaque fois un condensateur pour la première et la seconde impulsions de stimulation.

10. Appareil de stimulation selon la revendication 5, **caractérisé en que** l'unité de commande est conçue pour la détermination autonome de la valeur de référence ou des caractéristiques morphologiques prédéfinies par autocalibrage.

11. Appareil de stimulation selon la revendication 10, **caractérisé en que** l'autocalibrage est effectué par l'enregistrement et la comparaison d'une pluralité de différences de temps par le fait que la plus grande différence de temps ou la valeur moyenne des plus grandes différences de temps est déterminée à partir de la majorité des différences de temps enregistrées par rapport à la valeur de référence.

12. Appareil de stimulation selon la revendication 10, **caractérisé en ce que** l'autocalibrage s'effectue par détermination de la différence de temps qui est attribuée à une impulsion de stimulation présentant la plus faible intensité de stimulation d'une pluralité d'impulsions de stimulation.

13. Appareil de stimulation selon la revendication 10, **caractérisé en ce que** l'autocalibrage s'effectue par la détermination de la valeur moyenne des différences de temps qui sont attribuées à un certain nombre d'impulsions de stimulation présentant les plus faibles intensités de stimulation à partir d'une pluralité d'impulsions de stimulation.

14. Appareil de stimulation selon la revendication 10, **caractérisé en ce que** l'autocalibrage s'effectue par détermination des caractéristiques morphologiques qui sont attribuées à une impulsion de stimulation présentant la plus faible intensité de stimulation d'une pluralité d'impulsions de stimulation.

15. Appareil de stimulation selon la revendication 10, **caractérisé en que** l'autocalibrage s'effectue par détermination de valeurs moyennes des caractéristiques morphologiques qui sont attribuées à un certain nombre d'impulsions de stimulation présentant les plus faibles intensités de stimulation à partir d'une pluralité d'impulsions de stimulation.

16. Appareil de stimulation selon la revendication 1, **caractérisé en ce que** l'appareil de stimulation est un pacemaker.
